# EUROPÄISCHE PATENTANMELDUNG

(11) **EP 2 072 031 A1**
(43) Veröffentlichungstag der Anmeldung: **24.06.2009**
(21) Anmeldenummer: 08021796.1
(22) Anmeldetag: 16.12.2008
(51) Int. Cl.: A61K 8/02, A61Q 17/04, A61Q 19/00, A61K 8/92

(54) **Paraffinfreier Lippenpflegestift**

(30) Priorität: 20.12.2007 DE 102007061897
(71) Anmelder: Hempel, Matthias, 90562 Heroldsberg (DE)
(72) Erfinder: Hempel, Matthias, 90562 Heroldsberg (DE)
(74) Vertreter: Bauerschmidt, Peter

(57) **Zusammenfassung**

Die Erfindung betrifft einen paraffinfreien Lippenpflegestift mit einer Temperaturstabilität von mindestens 45° C. Er umfasst Bienenwachs oder ein Bienenwachs/Pflanzenwachs-Gemisch einerseits und Öl andererseits. Das Öl hat bezogen auf das Gesamtgewicht einen Ölanteil von mindestens 69 % und ist ein Pflanzenöl mit Ausnahme von Rizinusöl. Außerdem umfasst das Öl zumindest auch eine schmelzpunkterhöhende Komponente in Form eines hydrierten Pflanzenöls.

## Beschreibung

Die Erfindung richtet sich auf einen paraffinfreien Lippenpflegestift enthaltend Bienenwachs und Öl.

Eine paraffinfreie kosmetische Zubereitung zur Lippenpflege in Form eines Lippengels ist z. B. bekannt aus DE 195 41968 A1. Weiterhin ist eine paraffinfreie Lippenstiftgrundmasse aus der DE 724 274 sowie eine paraffinfreie Lippenpomade aus der CH 551 195 bekannt.

Lippenstifte nach dem Stand der Technik mit einem Gehalt an Paraffin und Bienenwachs sind bekannt aus "Kosmetik, Entwicklung, Herstellung und Anwendung kosmetischer Mittel", Seite 105, Georg Thieme Verlag, Stuttgart-New York, 1988.

Lippenpflegestifte enthalten nur geringfügige Mengen an Pigmenten oder gar keine Pigmente. Sofern eine geringe Menge von Pigmenten enthalten ist, üben diese keinen wesentlichen Einfluss auf Struktur oder Stabilität der Stifte aus. Gut formulierte Lippenpflegestifte zeichnen sich durch einen angenehmen Auftrag auf die Lippen aus und wirken durch die enthaltenen natürlichen Öle pflegend.

Lippenpflegestifte weisen in der Regel mindestens ein hochschmelzendes Wachs auf. Sowohl beim Aufschmelzen der Pflegestiftrezeptur als auch beim Gießen der Stifte, muss man mindestens die Schmelztemperatur des höchstschmelzenden Wachses erreichen, das heißt die Verarbeitungstemperatur liegt üblicherweise bei mindestens 80° C. Dies bedeutet den Anfall hoher Energiekosten. Die Erfahrung zeigt auch, dass man ebenfalls beim Gießen der Stifte diese Temperatur einhalten muss, da anderenfalls die Erweichungstemperatur unter den erwünschten 45° C liegen würde. Auch die Minenstabilität wäre geringer, da die hochschmelzenden Wachse offensichtlich beim Abkühlen nur dann zur erwünschten Formstabilität auskristallisieren, wenn sie vorher vollständig aufgeschmolzen waren.

Aufgrund von in jüngerer Zeit durchgeführten Untersuchungen und Veröffentlichungen befürchten manche Verbraucher gesundheitsschädigende Einflüsse von Paraffinen, welche herkömmlicherweise für derartige Stifte verwendet werden. Man hat deshalb Rezepturen ohne Paraffin entwickelt. Bekannte Lippenpflegestifte der gattungsgemäßen Art enthalten eine Vielzahl unterschiedlicher Komponenten und sind aufwändig in der Herstellung.

Hiervon ausgehend liegt der Erfindung die Aufgabe zugrunde, einen derartigen Lippenpflegestift so auszugestalten, dass er möglichst wenig unterschiedliche Komponenten in wesentlichen Anteilen enthält, dass er bei niedrigeren Temperaturen zu verarbeiten ist und hierdurch der Energieaufwand reduziert und die Taktfrequenz beim Gießen in der Herstellung erhöht werden kann, so dass eine kostengünstige Herstellung realisierbar ist.

Diese Aufgabe wird erfindungsgemäß durch einen Lippenpflegestift gemäß Anspruch 1 gelöst. Der erfindungsgemäße Lippenpflegestift hat eine Temperaturstabilität von mindestens 45° C und umfasst Bienenwachs (Cera Alba) oder ein Bienenwachs/Pflanzenwachs-Gemisch einerseits und Öl andererseits, wobei das Öl bezogen auf das Gesamtgewicht einen Ölanteil von mindestens 69 % hat, das Öl ein Pflanzenöl mit Ausnahme von Rizinusöl ist, und das Öl zumindest auch eine schmelzpunkterhöhende Komponente in Form eines hydrierten Pflanzenöls umfasst.

Insbesondere kann es vorgesehen sein, dass das Pflanzenöl zumindest eines aus der Gruppe von Sojaöl und hydriertem Baumwollsaatöl umfasst.

Vor allem kann vorgesehen sein, dass das Pflanzenöl eine Mischung von Sojaöl und hydriertem Baumwollsaatöl ist.

Es wurde gefunden, dass eine derartige Zusammensetzung sich überraschenderweise bei Temperaturen von ca. 65° C (dem Schmelzpunkt von Bienenwachs) herstellen lassen, die um ca. 20° C niedriger liegen als herkömmliche Temperaturen. Trotzdem wird die gewünschte Konsistenz im Endzustand erreicht und ein partielles Auskristallisieren zuverlässig vermieden und eine Temperaturstabilität von mindestens 45° C erzielt. Letzteres ist auch insofern bemerkenswert, da ein vergleichsweise hoher Ölanteil vorgesehen ist. Das hydrierte Pflanzenöl setzt den Schmelzpunkt des Ölanteils soweit herauf, dass die Lippenpflegestiftsubstanz insgesamt einen Schmelzpunkt über 45° C hat und somit bis zu dieser Temperatur - wie gefordert - stabil ist. Das verwendete hydrierte Pflanzenöl hat einen Schmelzpunkt von insbesondere höchstens 45° C und von vorzugsweise zwischen 37° C und 41° C. Der Schmelzpunkt von hydriertem Baumwollsaatöl liegt beispielsweise bei etwa 39° C.

Gemäß einer weiteren besonderen Ausgestaltung ist ein Anteil des nichthydrierten Pflanzenöls um den Faktor 6 bis 10 größer als ein Anteil des hydrierten Pflanzenöls. Dadurch erreicht man zum einen, dass der Schmelzpunkt des Ölanteils insgesamt zwar etwas, aber zum anderen auch nicht zu stark angehoben wird.

Besonders vorteilhafte Eigenschaften werden dadurch erreicht, dass der Wachsanteil, vorzugsweise der Bienenwachsanteil, bei 18 % bis 22 %, insbesondere bei ca. 20 %, und der Ölanteil bei 77 % bis 81 %, insbesondere bei ca. 80 % liegt.

Außer diesen die Grundeigenschaften des Lippenstifts bestimmenden wesentlichen Anteilen können in kleinen Mengen medizinisch oder kosmetisch wirksame Zusätze vorgesehen sein.

Gemäß einer weiteren besonderen Ausgestaltung hat jede einzelne der Komponenten des Lippenpflegestifts jeweils einen Schmelzpunkt von höchstens 65° C. Damit muss die Mischung aller Ausgangssubstanzen (= einzelne Komponenten des Lippenpflegestifts) auf nur diese 65° C erwärmt werden, um alle Bestandteile zumindest einmal vollständig aufzuschmelzen. Eine Erwärmung auf nur 65° C ist besonders energieeffizient, da bisherige Mischungen deutlich stärker erwärmt werden müssen. Da der Schmelzpunkt der verwendeten Pflanzenöle ohnehin niedriger liegt, wird der genannte Wert von 65° C maßgeblich durch das Bienenwachs bestimmt, dessen Schmelzpunkt in etwa bei dieser Temperatur liegt.

Die Verwendung derartiger pflanzlicher, also natürlicher Öle bzw. Ölmischungen für kosmetische Zwecke ist ebenso wie der Einsatz von Bienenwachs und ggf. einem Pflanzenwachsanteil an sich bekannt. Die Besonderheit der erfindungsgemäßen Lösung liegt darin, dass überraschenderweise ein Lippenstift mit sowohl sehr guten kosmetischen Eigenschaften als auch vorteilhaften mechanischen Eigenschaften mit im Wesentlichen nur drei Komponenten herstellbar ist.

Die Vorteile der erfindungsgemäßen Lösung liegen in einer Energieeinsparung aufgrund der niedrigeren Gießtemperatur von insbesondere etwa 65° C, der Vermeidung von Beschädigungen von temperaturempfindlichen Beimischungen geringen Veraibeitungskosten aufgrund des Einsatzes von im Wesentlichen nur zwei bzw. drei Basisstoffen (nämlich Bienenwachs und Pflanzenöl sowie hydriertes Pflanzenöl), höheren Taktzahlen beim Gießen aufgrund einer geringeren Abkühldauer und der Verwendung von gesundheitlich unschädlichen pflanzlichen Rohstoffen und Bienenwachs.

Nachfolgend wird die Erfindung anhand zweier bevorzugter Ausführungsbeispiele erläutert:

### Beispiel 1:

| | |
|---|---|
| Bienenwachs (Cera Alba) | 20% |
| Pflanzenöle | 71% |
| hydrierte Pflanzenöle | 8% |
| Pflegestoffe/Lichtschutzfilter | 1% |

### Beispiel 2:

| | |
|---|---|
| Bienenwachs/Pflanzenwachs-Gemisch | 20% |
| Pflanzenöle | 69% |
| hydrierte Pflanzenöle | 10% |
| Pflegestoffe/Lichtschutzfilter | 1% |

## Patentansprüche

1. Paraffinfreier Lippenpflegestift mit einer Temperaturstabilität von mindestens 45°C umfassend Bienenwachs oder ein Bienenwachs/Pflanzenwachs-Gemisch einerseits und Öl andererseits, wobei
a) das Öl bezogen auf das Gesamtgewicht einen Ölanteil von mindestens 69 % hat,
b) das Öl ein Pflanzenöl mit Ausnahme von Rizinusöl ist, und
c) das Öl zumindest auch eine schmelzpunkterhöhende Komponente in Form eines hydrierten Pflanzenöls umfasst.

2. Lippenpflegestift nach Anspruch 1, **dadurch gekennzeichnet, dass** das Pflanzenöl zumindest eines aus der Gruppe von Sojaöl, Baumwollsaatöl und Olivenöl umfasst.

3. Lippenpflegestift nach Anspruch 1 oder 2, **dadurch gekennzeichnet, dass** als Pflanzenöl eine Mischung von Sojaöl und hydriertem Baumwollsaatöl vorgesehen ist.

4. Lippenpflegestift nach einem der Ansprüche 1 bis 3, **dadurch gekennzeichnet, dass** ein Anteil des nichthydrierten Pflanzenöls um den Faktor 6 bis 10 größer ist als ein Anteil des hydrierten Pflanzenöls.

5. Kosmetikstift nach einem der Ansprüche 1 bis 4, **dadurch gekennzeichnet, dass** ein Wachsanteil bei 18 % bis 22 % und der Ölanteil bei 77 % bis 81 % liegt.

6. Kosmetikstift nach einem der Ansprüche 1 bis 5, **dadurch gekennzeichnet, dass** jede einzelne der Komponenten des Kosmetikstifts jeweils einen Schmelzpunkt von höchstens 65° C hat.

7. Kosmetikstift nach einem der Ansprüche 1 bis 6, **dadurch gekennzeichnet, dass** das hydrierte Pflanzenöl einen Schmelzpunkt von höchstens 45° C hat.
